# EUROPÄISCHE PATENTSCHRIFT

(11) **EP 1 201 190 B1**
(45) Veröffentlichungstag und Bekanntmachung des Hinweises auf die Patenterteilung: **31.03.2004**
(21) Anmeldenummer: 01125990.0
(22) Anmeldetag: 30.10.2001
(51) Int. Cl.: A61B 17/04

(54) **Vorrichtung zum Hindurchführen wenigstens zweier Nähfäden durch eine Wandung, insbesondere einer Arterie**
Device for guiding at least two sutures through a wall, in particular an arterial wall
Appareil pour guider au moins deux sutures à travers une paroi, en particulier d'une artère

(30) Priorität: 31.10.2000 DE 10053884
(43) Veröffentlichungstag der Anmeldung: 02.05.2002
(73) Patentinhaber: Medi-Globe GmbH, 83101 Rohrdorf-Achenmühle (DE)
(72) Erfinder: Ischinger, Thomas, Prof. Dr. med., 81925 München (DE); Boehlke, Raimar, Dipl.-Ing., 83064 Raubling (DE); Ehrl, Thomas, Dipl.-Ing., 83209 Prien am Chiemsee (DE)
(74) Vertreter: Nätebusch, Roderich

(56) Entgegenhaltungen:
- WO-A-01/17434
- US-A- 5 304 184
- US-A- 5 417 699
- US-A- 5 527 322
- US-A- 5 860 990

## Beschreibung

Das deutsche Patent DE 199 42 951.0-C1 bezieht sich auf eine Vorrichtung zum Hindurchführen wenigstens zweier Nähfäden durch eine Wandung einer Hülle, eines Ballons oder einer Fläche, insbesondere einer Arterie eines Individuums nahe des Randbereiches einer darin vorhandenen, gegebenenfalls durch Ein- und/oder Freischneiden gebildeten Öffnung und zum Zurückziehen der durch die betreffende Wandung hindurchgeführten Nähfädenenden aus der genannten Öffnung, mit einer stabförmigen Fadenführungseinrichtung, in der die an Nadeln befestigten Nähfäden in Führungs- bzw. Aufnahmeöffnungen derart geführt sind, dass sie mit der betreffenden Fadenführungseinrichtung durch die Wandung der betreffenden Hülle oder des betreffenden Ballons oder Blutgefäßes hindurchführbar und aus der genannten Öffnung wieder derart zurückziehbar sind , dass durch Zusammenziehen der Nähfädenenden außerhalb der betreffenden Öffnung diese verschließbar ist,
wobei die Fadenführungseinrichtung in ihrer Längsrichtung ein hinteres Fadenzuführungsteil, ein vorderes Fadenaufnahmeteil und ein dazwischen liegendes mittleres Fadenfreigabe-/Fadenklemmteil enthält
und wobei das genannte mittlere Fadenfreigabe-/Fadenklemmteil zumindest relativ zu dem vorderen Fadenaufnahmeteil verdrehbar ist und einen solchen Querschnitt aufweist, dass es in zumindest einer Drehstellung die Einführung der von dem hinteren Fadenzuführungsteil zugeführten Nähfäden in in dem vorderen Fadenaufnahmeteil freiliegende Aufnahmeöffnungen ermöglicht und in einer von der genannten Drehstellung verschiedenen Drehposition die in den betreffenden Aufnahmeöffnungen zusammen mit den Nadeln aufgenommenen Nähfäden für ein Herausziehen der gesamten Fadenführungseinrichtung aus den genannten Öffnungen heraus festzuhalten gestattet.

Obwohl die betreffende Vorrichtung zufriedenstellend arbeitet, besteht zuweilen der Wunsch, die durch das verwendete mittlere Fadenfreigabe-/Fadenklemmteil erzielte Spannung der erwähnten Wandung einer Hülle, eines Ballons oder einer Fläche, insbesondere einer Arterie eines Individuums nahe des Randbereiches einer darin vorhandenen, gegebenenfalls durch Ein- und/oder Freischneiden gebildeten Öffnung noch zu steigern. Dies trifft insbesondere für den Fall zu, dass die betreffende Wandung durch eine Arterie eines Individuums gebildet ist, in der das erwähnte mittlere Fadenfreigabe-/Fadenklemmteil der betreffenden Vorrichtung gewissermaßen als Wundrandspanner wirkt.

Der Erfindung liegt demgemäß die Aufgabe zugrunde, einen Weg zu zeigen, wie bei einer Vorrichtung der eingangs genannten Art auf besonders wirksame Weise die durch die Wandung der erwähnten Hülle, des erwähnten Ballons oder der erwähnten Fläche, insbesondere einer Arterie eines Individuums zusammen mit Nähfäden hindurchzuführenden Nadeln in derart sicheren Abstand vom jeweiligen Wandungsrand hindurchgeführt werden können, dass es nicht zum Einreißen der jeweiligen Wandung kommt.

Gelöst wird die vorstehend aufgezeigte Aufgabe bei einer Vorrichtung der eingangs genannten Art erfindungsgemäß dadurch, dass das genannte mittlere Fadenfreigabe-/Fadenklemmteil in einer Achsrichtung derart aufweitbar ist, dass dadurch die im Bereich des betreffenden mittleren Fadenfreigabe-/Fadenklemmteiles befindliche Wandung der Hülle, des Ballons oder einer Fläche, insbesondere einer Gefäßwand einer Arterie eines Individuums eine Spannung erfährt, auf Grund derer die quer zu dieser Spannrichtung vorhandenen Randbereiche der betreffenden Hüllen-, Ballon- oder Gefäßwandung sich in Richtung aufeinander zu zusammenziehen.

Durch die in der zuvor genannten Richtung zusammengezogenen Randbereiche der betreffenden Hüllen-, Ballon- oder Flächen- bzw. Gefäßwandung aufgrund der erwähnten Spannung liegt der jeweilige Hüllen-, Ballon- oder Flächen- bzw. Arterienrandbereich am Außenumfang bzw. Randbereich des Fadenfreigabe-/Fadenklemmteiles besonders sicher und damit fest an, so dass die Nadeln mit ihren Nähfäden dort durch die betreffenden Wandungsrandbereiche in sicherem Abstand hindurchgeführt werden können, ohne dass es zu einem Einreißen der betreffenden Randbereiche kommt. Dies ist insbesondere in Fällen von kleinen bis sehr kleinen Öffnungen in einer Gefäßwand von Vorteil, bei der die Durchstichstellen der erwähnten Nadeln durch die betreffenden Gefäßwandbereiche sehr klein sind, beispielsweise in der Größenordnung von 0,5 bis 1,5 mm liegen. Von Vorteil ist es ferner, dass der Arbeitsdurchmesser der das mittlere Fadenfreigabe-/Fadenklemmteil sowie das Fadenzuführungsteil und das Fadenaufnahmeteil enthaltenden Vorrichtung gemäß dem Hauptpatent kleiner als bisher gewählt werden kann. Durch ein Aufweiten des betreffenden mittleren Fadenfreigabe-/Fadenklemmteiles entsprechend dem jeweiligen Anwendungsfall steht somit eine universell einsetzbare Vorrichtung zum Verschließen unterschiedlich großer Öffnungen in einer Wandung einer Hülle, eines Ballons oder einer Fläche, insbesondere einer Arterie eines Individuums zur Verfügung.

Zweckmäßigerweise ist das mittlere Fadenfreigabe-/Fadenklemmteil mechanisch und/oder elektrisch und/oder pneumatisch/ hydraulisch aufweitbar. Dadurch läßt sich das betreffende mittlere Fadenfreigabe-/Fadenklemmteil in vorteilhafter Weise unterschiedlichsten Wünschen bzw. Bedürfnissen entsprechend aufweiten.

Von Vorteil ist es ferner, das mittlere Fadenfreigabe-/Fadenklemmteil dadurch in der genannten einen Achsrichtung aufzuweiten, dass zumindest ein in dieser Achsrichtung liegendes Randteil des betreffenden Fadenfreigabe-/Fadenklemmteiles ausfahrbar ist. Dies ermöglicht in vorteilhafter Weise eine besonders einfache Konstruktion des mittleren Fadenfreigabe-/Fadenklemmteiles für ein Ausfahren.

Von Vorteil ist es ferner, das mittlere Fadenfreigabe-/Fadenklemmteil in einen mittleren Teil und in zwei angrenzende Randteile aufzuteilen, die relativ zu dem betreffenden mittleren Teil bewegbar sind. Hierdurch läßt sich auf besonders einfache Weise ein symmetrisch geschaltetes Fadenfreigabe-/Fadenklemmteil für ein Ausfahren seiner Randbereiche realisieren.

Zweckmäßigerweise ist das jeweilige Randteil des mittleren Fadenfreigabe-/Fadenklemmteiles mit einer Drück- und Zieheinrichtung verbunden, durch deren Betätigung das jeweilige Randteil relativ zu dem mittleren Teil verschiebbar ist. Auf diese Weise läßt sich das Ausfahren des jeweiligen Randteiles auf besonders einfache Weise realisieren.

Es ist aber auch möglich, dass das mittlere Fadenfreigabe-/Fadenklemmteil zwei Klemmteile aufweist, die in einem inaktiven Zustand von einem Aufnahmerohr aufgenommen sind und die in diesem durch eine Krafteinrichtung, insbesondere eine Feder einem nach außen gerichteten Bewegungsdruck ausgesetzt sind und die nach Zurückziehen des betreffenden Aufnahmerohres in einen aktiven Zustand voneinander weg drückbar sind. Diese Maßnahme bringt den Vorteil mit sich, dass ohne eine gesonderte Drückeinrichtung ausgekommen werden kann, um die erwähnten beiden Klemmteile für ein Aufweiten des mittleren Fadenfreigabe-/Fadenklemmteiles auszufahren.

Zweckmäßigerweise sind die beiden gerade erwähnten Klemmteile dabei mittels einer Zieheinrichtung in Richtung aufeinander zu zusammenführbar. Dadurch können auf besonders einfache Weise die beiden erwähnten Klemmteile wieder in den inaktiven Zustand zurückgebracht werden.

Gemäß einer weiteren zweckmäßigen Ausgestaltung der Erfindung sind die genannten Randteile mit dem betreffenden mittleren Teil über pneumatische/hydraulische Kammern verbunden und durch pneumatische/hydraulische Wirkung relativ zu dem genannten mittleren Teil bewegbar. Hierdurch ergibt sich der Vorteil einer besonders einfachen Konstruktion für eine Relativbewegung der erwähnten Randteile zu dem genannten mittleren Teil.

Gemäß einer weiteren zweckmäßigen Ausgestaltung der Erfindung sind die genannten Randteile mittels einer Kulissensteuereinrichtung relativ zueinander bewegbar sind. Dies führt zu einer besonders stabilen Konstruktion, um die erwähnten Randteile relativ zu dem genannten mittleren Teil verschieben zu können.

Als besonders vorteilhaft hat es sich herausgestellt, das mittlere Fadenfreigabe/Fadenklemmteil in seinem Außenbereich für eine Anlage am Randbereich der genannten Hüllen-, Ballonoder Flächen- insbesondere Gefäßwand konkav auszubilden. Dadurch liegt die jeweilige Hüllen-, Ballon- oder Flächen- bzw. Gefäßwand sicher am Außenumfangsbereich des Fadenfreigabe-/Fadenklemmteiles an und kann nicht ohne weiteres aus diesem Anlagebereich austreten.

Anhand von Zeichnungen wird die Erfindung nachstehend beispielsweise näher erläutert.
- Fig. 1: zeigt in einer schematischen Ansicht von der im obengenannten Patent dargestellten und beschriebenen Vorrichtung lediglich ein Fadenzuführungsteil, ein Fadenaufnahmeteil und ein dazwischen vorgesehenes Fadenfreigabe-/Fadenklemmteil.
- Fig. 2: zeigt eine Schnittansicht des in Fig. 1 schematisch angedeuteten Fadenfreigabe-/Fadenklemmteiles längs der in Fig. 1 eingetragenen Schnittebene X-X.
- Fig. 3: zeigt in einer Schnittansicht eine andere Ausführungsform des betreffenden Fadenfreigabe-/Fadenklemmteiles in einer inaktiven Position.
- Fig. 4: zeigt die in Fig. 3 dargestellte Anordnung in einem aktiven Zustand.
- Fig. 5 und 6: zeigen in einer schematischen Darstellung eine noch weitere Ausführungsform des erwähnten Fadenfreigabe-/Fadenklemmteiles.
- Fig. 7: zeigt in einer weiteren schematischen Darstellung eine noch weitere alternative Ausführungsform des betreffenden Fadenfreigabe-/Fadenklemmmteiles.

Bevor auf die in den Zeichnungen schematisch dargestellten Einzelheiten eingegangen wird, sei vorab grundsätzlich bemerkt, dass hier lediglich diejenigen Vorrichtungsteile erläutert werden, mit denen sich die vorliegende Erfindung von den im Hauptpatent erfaßten Vorrichtungen unterscheidet.

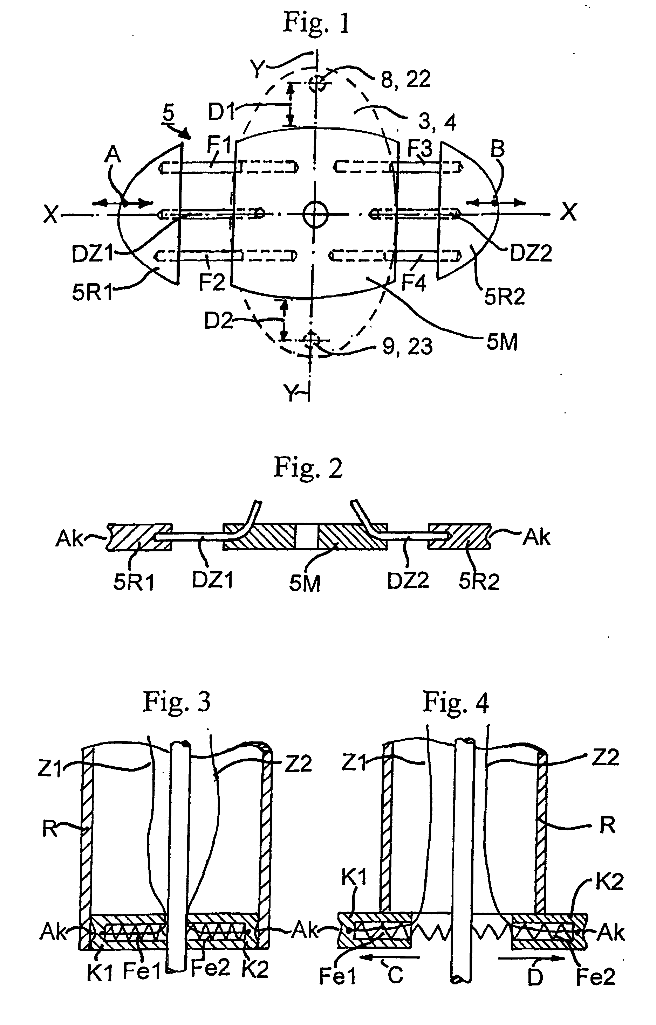

In Fig. 1 sind schematisch ein Fadenzuführungsteil 3, ein Fadenaufnahmeteil 4 und ein mittleren Fadenfreigabe-/Fadenklemmteil 5 angedeutet, wie sie in den verschiedenen Vorrichtungen vorhanden sind, die im DE 199 42 951.0-C1 erfaßt sind. Das mittlere Fadenfreigabe-/Fadenklemmteil 5, befindet sich gemäß Fig. 1 in der Fadenfreigabeposition, in der Nadeln mit Nähfäden durch die angedeuteten Öffnungen 8, 22 bzw. 9, 23 des Fadenzuführungsteiles 3 und des Fadenaufnahmeteiles 4 bewegt und damit durch den Randbereich einer Öffnung in einem zwischen diesen Teilen 3 und 4 befindlichen Hüllen-, Ballon- oder Flächenteil, insbesondere einer Arterie eines Individuums hindurchgeführt werden können. Die betreffenden Hüllen-, Ballonoder Flächen- bzw. Arterienrandbereich liegen dabei an dem Außenumfang des Fadenfreigabe-/Fadenklemmteiles 5 derart an, dass zwischen dem jeweiligen Randbereich und der Mitte der jeweiligen Nadel ein Abstand D1 bzw. D2 gegeben ist. Es sei hier noch angemerkt dass das Fadenzuführungsteil 3, das Fadenaufnahmeteil 4 und das mittlere Fadenfreigabe-/Fadenklemmteil 5 jeweils als einen ovalförmigen Querschnitt aufweisende Teile angedeutet sind; die Erfindung ist hierauf allerdings nicht beschränkt. So können die betreffenden Teile beispielsweise jeweils einen kreisförmigen Querschnitt aufweisen; das erwähnte Fadenfreigabe-/Fadenklemmteil 5 kann im übrigen eine längliche Querschnittsform aufweisen, wie dies im Hauptpatent gezeigt ist.

Um den jeweiligen Hüllen-, Ballon- oder Flächen- bzw. Arterienrandbereich am Außenumfang bzw. Randbereich des Fadenfreigabe-/Fadenklemmteiles 5 besonders sicher anliegen zu lassen, ist gemäß der vorliegenden Erfindung vorgesehen, das betreffende Fadenfreigabe-/Fadenklemmteil 5 in einer Achsrichtung, das ist in Richtung X-X gemäß Fig. 1 aufzuweiten. Diese Achsrichtung verläuft gemäß Fig. 1 von links nach rechts und damit rechtwinklig zu der Achse Y-Y, auf der die Öffnungen 8, 22 bzw. 9, 23 liegen. Dieses Aufweiten des Fadenfreigabe-/Fadenklemmteiles 5 erfolgt gemäß Fig. 1 symmetrisch um die Mittelachse des betreffenden Teiles 5 in Richtung der dort eingetragenen Pfeile A und B. Durch dieses Aufweiten des Fadenfreigabe-/Fadenklemmteiles 5, wirkt dieses Teil 5 im Falle des Einsatzes der betreffenden Vorrichtung zum Verschließen einer in einer Gafäßwand, wie einer Arterie vorhandenen Öffnung zum Spannen dieser Öffnung und damit als Wundrandspanner. Dadurch erfährt die im Bereich des betreffenden mittleren Fadenfreigabe-/Fadenklemmteiles 5 befindliche Wandung der Hülle, des Ballons oder einer Fläche bzw. einer Gefäßwand, insbesondere einer Arterie eines Individuums, eine Spannung, aufgrund derer die quer zu dieser Spannrichtung (X-X), also in der Richtung Y-Y vorhandenen Randbereiche der betreffenden Hüllen-, Ballon- oder Flächen- bzw. Gefäßwand sich in Richtung aufeinander zu zusammenziehen. Dies heißt, dass sich die betreffende Hüllen-, Ballon- oder Flächen- bzw. Gefäßwand dadurch ganz eng an den in Querrichtung Y-Y zu der betreffenden Spannrichtung X-X verlaufenden Bereich des Fadenfreigabe-/Fadenklemmteiles 5 anlegt. Dies stellt somit sicher, dass mit den im Bereich der erwähnten Öffnungen 8, 22 bzw. 9, 23 geführten Nadeln kein ausgefranster Hüllen-, Ballon- oder Gefäßwandbereich durchstochen wird, sondern tatsächlich ein glatter Rand, womit ein Einreißen des jeweiligen Randbereiches der Hüllen-, Ballonoder Gefäßwand im Bereich der jeweiligen Nadeleinstechstelle vermieden ist. Eine entsprechende Wirkung ergibt sich übrigens auch in den Fällen, dass das Fadenfreigabe-/Fadenklemmteil 5 längs einer oder mehrerer Achsen aufgeweitet wird, die zwar quer zur Achse Y-Y verlaufen, dieser gegenüber jeweils einen von 90° verschiedenen Winkelwert aufweisen.

Fig. 1 zeigt dabei schematisch, dass das erwähnte Aufweiten des mittleren Fadenfreigabe-/Fadenklemmteiles 5 dadurch möglich ist, dass dieses Teil 5 in ein mittleres Teil 5M und in zwei angrenzende Randteile 5R1 und 5R2 aufgeteilt ist. Die Randteile 5R1, 5R2 sind mit dem mittleren Teil 5M über Führungselemente F1, F2 bzw. F3, F4 verbunden. Diese Randteile 5R1, 5R2 können, wie aus der in Fig. 2 dargestellten schematischen Schnittansicht hervorgeht, mechanisch von Drück- und Ziehdrähten DZ1, DZ2 relativ zu dem mittleren Teil 5M bewegt werden. An dieser Stelle sei angemerkt, dass die Anordnung grundsätzlich auch so getroffen sein kann, dass lediglich eines der gerade erwähnten Randteile 5R1, 5R2 des Fadenfreigabe-/Fadenklemmteiles 5 in der erwähnten Achsrichtung ausfahrbar ist, das heißt in der in Fig. 1 angegebenen Richtung X-X.

Eine andere alternative Ausführungsform des mittleren Fadenfreigabe-/Fadenklemmteiles gemäß der Erfindung ist in Fig. 3 und 4 schematisch angedeutet. Danach weist das mittlere Fadenfreigabe-/Fadenklemmteil 5 zwei Klemmteile K1, K2 auf, die in einem inaktiven Zustand von einem Aufnahmerohr R aufgenommen sind und in diesem durch eine Krafteinrichtung, insbesondere durch eine Feder Fe1 bzw. Fe2 einem nach außen gerichteten Bewegungsdruck ausgesetzt sind und die nach Zurückziehen des betreffenden Aufnahmerohres R in einen aktiven Zustand in Richtung der Pfeile C und D voneinander weg drückbar sind. Die jeweilige Feder Fe1, Fe2 ist hier vorzugsweise eine Druckfeder. Die betreffenden Verhältnisse sind in Fig. 3 und 4 klar veranschaulicht. Um die erwähnten beiden Klemmteile K1, K2 wieder zusammenzuführen, wie dies in Fig. 3 veranschaulicht ist, ist vorzugsweise eine Zieheinrichtung in Form von Ziehdrähten Z1, Z2 vorgesehen, mit deren Hilfe die Klemmteile K1, K2 wieder zusammengezogen werden können, so dass anschließend das erwähnte Aufnahmerohr R wieder über die Klemmteile K1, K2 geschoben werden kann.

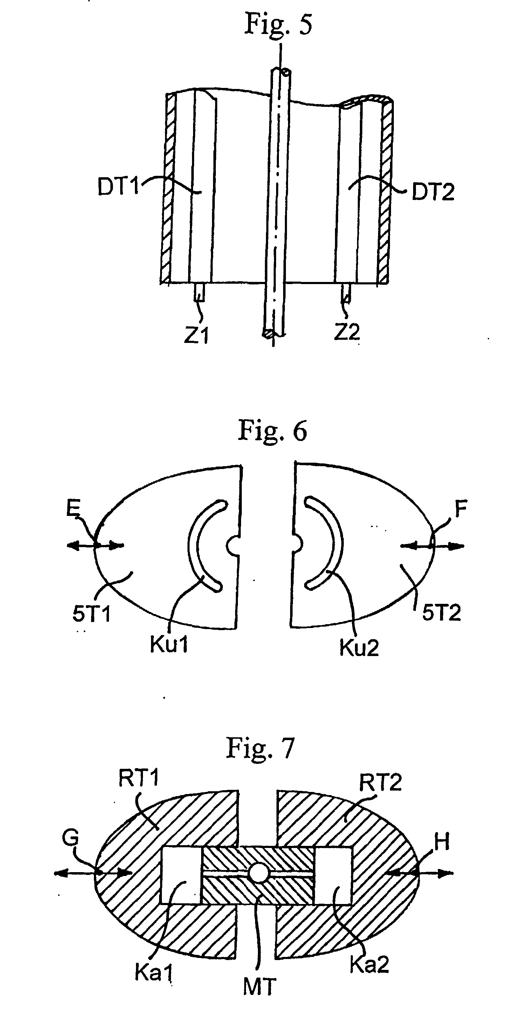

Eine weitere Modifikation des mittleren Fadenfreigabe-/Fadenklemmteiles gemäß der Erfindung ist in Fig. 5 und 6 veranschaulicht. In beiden Fig. 5 und 6 ist eine Kulissensteuereinrichtung dargestellt, bestehend aus zwei Drehteilen DT1, DT2, wie in Fig. 5 veranschaulicht ist, und einem das eigentliche mittlere Fadenfreigabe-/Fadenklemmteil 5 bildenden Randteilen 5T1 und 5T2, in denen sich jeweils eine Kulisse Ku1 bzw. Ku2 befindet. In diese Kulissen greifen die Drehteile DT1, DT2 mit entsprechenden Zapfen Z1 bzw. Z2 ein. Durch Verdrehen der Drehteile DT gemäß Fig. 5 können die beiden Randteile 5T1 und 5T2 des in Fig. 6 dargestellten Fadenfreigabe-/Fadenklemmteiles 5 in Richtung ihrer Längsachse voneinander weg bzw. aufeinander zu bewegt werden, wie dies durch Pfeile E und F in Fig. 6 angedeutet ist.

In Fig. 7 ist ein weiteres Ausführungsbeispiel der Fadenfreigabe-/Fadenklemmeinrichtung gemäß der Erfindung dargestellt. Die Fig. 7 zeigt dabei ein Fadenfreigabe-/Fadenklemmteil 5, dessen Randteile RT3, RT4 mit einem mittleren Teil MT über pneumatische/hydraulische Kammern KA1 bzw. KA2 verbunden sind und durch pneumatische und/oder hydraulische Wirkung relativ zu dem genannten mittleren Teil MT bewegbar sind, wie dies in Fig. 7 durch Pfeile G und H angedeutet ist. Das entsprechende Druck- bzw. Hydraulikmittel kann dabei beispielsweise von einer mittleren Öffnung des mittleren Teiles MT durch kleine Rohrleitungen, wie sie in Fig. 7 angedeutet sind, in die betreffenden Kammern Ka1 bzw. Ka2 eingeleitet bzw. aus diesen herausgeführt werden.

Im Zusammenhang mit den vorstehend anhand der Zeichnungen erläuterten Ausführungsbeispiele dürfte ersichtlich geworden sein, dass das mittlere Fadenfreigabe-/Fadenklemmteil 5 jeweils mechanisch oder pneumatisch bzw. hydraulisch aufweitbar ist. Es dürfte einzusehen sein, dass diese mechanische bzw. pneumatische/hydraulische Aufweitung auch in Kombination miteinander erfolgen kann und dass auch entweder allein oder zusätzlich zu solchen Aufweitungsmaßnahmen eine elektrische Aufweitung erfolgen kann. Diese kann beispielsweise mit Hilfe von piezoelektrischen Aktoren erfolgen, welche das jeweilige Randteil relativ zum jeweiligen mittleren Teil des Fadenfreigabe-/Fadenklemmteiles 5 zu verschieben gestatten.

Im übrigen sei noch darauf hingewiesen, dass das mittlere Fadenfreigabe-/Fadenklemmteil 5 jeweils in seinem Außen- bzw. Randbereich vorzugsweise für eine Anlage am Randbereich der erwähnten Hüllen-, Ballon- oder Flächen- bzw. Gefäßwand konkav ausgebildet ist, wie dies in den Fig. 2, 3 und 4 besonders deutlich veranschaulicht ist. Es versteht sich, dass eine solche konkave Ausnehmung Ak selbstverständlich auch bei allen anderen Ausführungsbeispielen gemäß der Erfindung zum Tragen kommen kann.

Abschließend sei noch angemerkt, dass der Aufweitungsweg des jeweiligen Fadenfreigabe-/Fadenklemmteiles 5 von der jeweiligen Anwendung der dieses Fadenfreigabe-/Fadenklemmteil enthaltenen Vorrichtung gemäß dem Hauptpatent abhängt. So ist es beispielsweise möglich, in einer Hülle bzw. einem Ballon das genannte Fadenfreigabe-/Fadenklemmteil 5 um beispielsweise 5 bis 10 mm bei einer Lochgröße von beispielsweise 10 bis 20 mm einer zu verschließenden Öffnung aufzuweiten. Im Falle des Einsatzes der betreffenden Vorrichtung zum Verschließen einer in einer Arterienwand eines Individuums gebildeten Öffnung von beispielsweise 3 bis 5 mm kann die Aufweitung des genannten Fadenfreigabe-/Fadenklemmteiles 5 beispielsweise 1 bis 2,5 mm betragen.

## Patentansprüche

1. Vorrichtung zum Hindurchführen wenigstens zweier Nähfäden durch eine Wandung einer Hülle, eines Ballons oder einer Fläche, insbesondere einer Arterie eines Individuums nahe des Randbereiches einer darin vorhandenen, gegebenenfalls durch Ein- und/oder Freischneiden gebildeten Öffnung und zum Zurückziehen der durch die betreffende Wandung hindurchgeführten Nähfädenenden aus der genannten Öffnung, mit einer stabförmigen Fadenführungseinrichtung, in der die an Nadeln befestigten Nähfäden in Führungs- bzw. Aufnahmeöffnungen (8, 22, 9, 23) derart geführt sind, dass sie mit der betreffenden Fadenführungseinrichtung durch die Wandung der betreffenden Hülle oder des betreffenden Ballons oder Blutgefäßes hindurchführbar und aus der genannten Öffnung wieder derart zurückziehbar sind , dass durch Zusammenziehen der Nähfädenenden außerhalb der betreffenden Öffnung diese verschließbar ist,
wobei die Fadenführungseinrichtung in ihrer Längsrichtung ein hinteres Fadenzuführungsteil (3), ein vorderes Fadenaufnahmeteil (4) und ein dazwischen liegendes mittleres Fadenfreigabe-/Fadenklemmteil (5), enthält
und wobei das genannte mittlere Fadenfreigabe-/Fadenklemmteil zumindest relativ zu dem vorderen Fadenaufnahmeteil verdrehbar ist und einen solchen Querschnitt aufweist, dass es in zumindest einer Drehstellung die Einführung der von dem hinteren Fadenzuführungsteil zugeführten Nähfäden in in dem vorderen Fadenaufnahmeteil freiliegende Aufnahmeöffnungen ermöglicht und in einer von der genannten Drehstellung verschiedenen Drehposition die in den betreffenden Aufnahmeöffnungen zusammen mit den Nadeln aufgenommenen Nähfäden für ein Herausziehen der gesamten Fadenführungseinrichtung aus den genannten Öffnungen heraus festzuhalten gestattet,
**dadurch gekennzeichnet,**
**dass** das genannte mittlere Fadenfreigabe-/Fadenklemmteil (5) in einer Achsrichtung (X-X) derart aufweitbar ist, dass dadurch die im Bereich des betreffenden mittleren Fadenfreigabe-/Fadenklemmteiles (5) befindliche Wandung der Hülle, des Ballons oder einer Fläche, insbesondere der Gefäßwand einer Arterie eines Individuums eine Spannung erfährt, auf Grund derer die quer zu dieser Spannrichtung vorhandenen Randbereiche der betreffenden Hüllen-, Ballon- oder Flächen- bzw. Gefäßwandung sich in Richtung aufeinander zu zusammenziehen.

2. Vorrichtung nach Anspruch 1, **dadurch gekennzeichnet, dass** das mittlere Fadenfreigabe-/Fadenklemmteil (5) mechanisch und/oder elektrisch und/oder pneumatisch/pneumatisch aufweitbar ist.

3. Vorrichtung nach Anspruch 2, **dadurch gekennzeichnet, dass** das mittlere Fadenfreigabe-/Fadenklemmteil (5) dadurch in der genannten einen Achsrichtung aufweitbar ist, dass zumindest ein in dieser Achsrichtung liegendes Randteil des betreffenden Fadenfreigabe-/Fadenklemmteiles ausfahrbar ist.

4. Vorrichtung nach Anspruch 2, **dadurch gekennzeichnet, dass** das mittlere Fadenfreigabe-/Fadenklemmteil (5) in einen mittleren Teil (5M) und in zwei angrenzende Randteile (5R1, 5R2) aufgeteilt ist, die relativ zu dem betreffenden mittleren Teil (5M) bewegbar sind.

5. Vorrichtung nach Anspruch 3 oder 4, **dadurch gekennzeichnet, dass** das jeweilige Randteil (5R1, 5R2) des mittleren Fadenfreigabe-/Fadenklemmteiles (5) mit einer Drück- und Zieheinrichtung (DZ1, DZ2) verbunden ist, durch deren Betätigung das jeweilige Randteil (5R1, 5R2) relativ zu dem mittleren Teil (5M) verschiebbar ist.

6. Vorrichtung nach Anspruch 3 oder 4, **dadurch gekennzeichnet, dass** das mittlere Fadenfreigabe-/ Fadenklemmteil (5) zwei Klemmteile (K1, K2) aufweist; die in einem inaktiven Zustand von einem Aufnahmerohr (R) aufgenommen sind und in diesem jeweils durch eine Krafteinrichtung, insbesondere eine Feder (Fe1, Fe2) einem nach außen gerichteten Bewegungsdruck ausgesetzt sind und die nach Zurückziehen des betreffenden Aufnahmerohres (R) in einem aktiven Zustand voneinander weg gedrückt sind.

7. Vorrichtung nach Anspruch 6, **dadurch gekennzeichnet, dass** die beiden Klemmteile (K1, K2) mittels einer Zieheinrichtung (Z1, Z2) in Richtung aufeinander zu zusammenführbar sind.

8. Vorrichtung nach Anspruch 4, **dadurch gekennzeichnet, dass** das die genannten Randteile (5T1, 5T2) mittels einer Kulissensteuereinrichtung (DT1, Z1, DT2, Z2, Ku1 Ku2) relativ zueinander bewegbar sind.

9. Vorrichtung nach Anspruch 4, **dadurch gekennzeichnet, dass** das die genannten Randteile (RT1, RT2) mit dem betreffenden mittleren Teil (MT) über pneumatische/hydraulische Kammern (Ka1, Ka2) verbunden und durch pneumatische/hydraulische Wirkung relativ zu dem genannten mittleren Teil (MT) bewegbar sind.

10. Vorrichtung nach einem der Ansprüche 1 bis 9, **dadurch gekennzeichnet, dass** das mittlere Fadenfreigabe-/Fadenklemmteil (5) in seinem Außenbereich für eine Anlage am Randbereich der genannten Hüllen-, Ballon- oder Flächen- bzw. Gefäßwand konkav (Ak) ausgebildet ist.

## Claims

1. An arrangement for guiding at least two sutures through a wall of a membrane, of a balloon or of a surface, in particular of an artery of an individual, in the vicinity of the edge region of an opening provided therein, and formed if appropriate by cutting in and/or cutting out, and for drawing back out of the abovementioned opening the suture ends guided through the relevant wall, having a shaft-like suture-guide device in which the sutures fastened on needles are guided in guide and/or accommodating openings (8, 22, 9, 23) such that, by way of the relevant suture-guide device, they can be guided through the wall of the relevant membrane or of the relevant balloon or blood vessel and drawn back out of the abovementioned opening again such that, by virtue of the suture ends being drawn together outside the relevant opening, the latter can be closed, it being the case that the suture-guide device, in its longitudinal direction, contains a rear suture-feed part (3), a front suture-accommodating part (4) and a central suture-release/suture-clamping part (5) located therebetween, and it being the case that the abovementioned central suture-release/suture-clamping part can be rotated at least relative to the front suture-accommodating part and has such a cross section that, in at least one rotary position, it allows the sutures fed from the rear suture-feed part to be introduced into accommodating openings exposed in the front suture-accommodating part and, in a rotary position differing from the abovementioned rotary position, it allows the sutures accommodated in the relevant accommodating openings together with the needles to be secured for drawing the entire suture-guide device out of the abovementioned openings,
**characterized in that** the abovementioned central suture-release/suture-clamping part (5) can be expanded in one axial direction (X-X) such that that wall of the membrane, of the balloon or of a surface, in particular of the vessel wall of an artery of an individual, which is located in the region of the relevant central suture-release/ suture-clamping part (5) is thus subjected to tensioning, on account of which those edge regions of the relevant membrane, balloon or surface or vessel wall which are provided transversely to said tensioning direction draw together toward one another.

2. The arrangement as claimed in claim 1, **characterized in that** the central suture-release/suture-clamping part (5) can be expanded mechanically and/or electrically and/or pneumatically/hydraulically.

3. The arrangement as claimed in claim 2, **characterized in that** the central suture-release/suture-clamping part (5) can be expanded in the abovementioned one axial direction **in that** at least one edge part of the relevant suture-release/suture-clamping part which is located in said axial direction can be moved in the outward direction.

4. The arrangement as claimed in claim 2, **characterized in that** the central suture-release/suture-clamping part (5) is divided up into a central part (5M) and into two adjacent edge parts (5R1, 5R2) which can be moved relative to the relevant central part (5M).

5. The arrangement as claimed in claim 3 or 4, **characterized in that** the respective edge part (5R1, 5R2) of the central suture-release/suture-clamping part (5) is connected to a pushing and pulling device (DZ1, DZ2), the actuation of which can displace the respective edge part (5R1, 5R2) relative to the central part (5M).

6. The arrangement as claimed in claim 3 or 4, **characterized in that** the central suture-release/suture-clamping part (5) has two clamping parts (K1, K2) which, in an inactive state, are accommodated by an accommodating tube (R) and, in the latter, are subjected to an outwardly directed movement pressure in each case by a force-exerting device, in particular a spring (Fe1, Fe2) and which, once the relevant accommodating tube (R) has been drawn back, are forced away from one another in an active state.

7. The arrangement as claimed in claim 6, **characterized in that** the two clamping parts (K1, K2) can be guided toward one another by means of a pulling device (Z1, Z2).

8. The arrangement as claimed in claim 4, **characterized in that** the abovementioned edge parts (5T1, 5T2) can be moved relative to one another by means of a slotted-guide control device (DT1, Z1, DT2, Z2, Ku1, Ku2).

9. The arrangement as claimed in claim 4, **characterized in that** the abovementioned edge parts (RT1, RT2) are connected to the relevant central part (MT) via pneumatic/hydraulic chambers (Ka1, Ka2) and can be moved relative to the abovementioned central part (MT) by pneumatic/ hydraulic action.

10. The arrangement as claimed in one of claims 1 to 9, **characterized in that**, in its outer region, the central suture-release/suture-clamping part (5) is designed concavely (Ak) for abutment against the edge region of the abovementioned membrane, balloon or surface or vessel wall.

## Revendications

1. Dispositif pour faire passer au moins deux fils de suture à travers la paroi d'une enveloppe, d'un ballon ou d'une surface, en particulier d'une artère d'un individu, à proximité de la bordure d'une ouverture qui y est présente et qui a éventuellement été réalisée par découpe et/ou libération, et pour extraire de ladite ouverture les extrémités du fil passées à travers la paroi concernée, qui comprend un dispositif de guidage de fil en forme de barre dans lequel les fils de suture fixés sur des aiguilles sont guidés dans des ouvertures (8, 22, 9, 23) de guidage et de réception de manière à pouvoir être passés à l'aide du dispositif de guidage de fil concerné à travers la paroi de l'enveloppe concernée, du ballon concerné ou du vaisseau sanguin et à être de nouveau extraits de ladite ouverture de manière à pouvoir fermer l'ouverture concernée en rassemblant les extrémités des fils de suture à l'extérieur de celle-ci,
le dispositif de guidage de fil contenant dans le sens de sa longueur une pièce arrière (3) d'amenée de fil, une pièce avant (4) de réception de fil et une pièce centrale (5), située entre les deux premières, de libération et de pinçage de fil,
ladite pièce centrale de libération et de pinçage de fil pouvant être tournée au moins par rapport à la pièce avant de réception de fil et présentant une section transversale telle qu'au moins dans une position de rotation, elle permet l'insertion du fil de suture amené par la pièce arrière d'amenée du fil jusque dans les ouvertures de réception libres situées dans la pièce avant de réception du fil et que dans une position de rotation différente de ladite position de rotation, elle permet de maintenir les fils de suture reçus en même temps que les aiguilles dans les ouvertures de réception concernées pour une extraction de l'ensemble du dispositif de guidage de fil hors desdites ouvertures,
**caractérisé en ce que** ladite pièce centrale (5) de libération et de pinçage de fil peut être déployée dans une direction axiale (X-X) de telle sorte qu'ainsi la paroi de l'enveloppe, du ballon ou d'une surface, en particulier la paroi d'une artère d'un individu, située dans la zone de la pièce centrale (5) de libération et de pinçage de fil concernée, subisse une contrainte suite à laquelle les bordures de la paroi concernée d'enveloppe, de ballon, ou de surface ou selon le cas de paroi de vaisseau situées transversalement par rapport à la direction de cette contrainte soient rapprochées l'une de l'autre.

2. Dispositif selon la revendication 1, **caractérisé en ce que** la pièce centrale (5) de libération et de pinçage du fil peut être déployée mécaniquement et/ou électriquement et/ou pneumatiquement/pneumatiquement.

3. Dispositif selon la revendication 2, **caractérisé en ce que** la pièce centrale (5) de libération et de pinçage de fil peut être déployée dans l'une desdites directions axiales grâce au fait qu'au moins une partie de bordure de la pièce de libération et de pinçage de fil concernée qui est située dans cette direction axiale peut être extraite.

4. Dispositif selon la revendication 2, **caractérisé en ce que** la pièce centrale (5) de libération et de pinçage de fil est divisée en une partie centrale (5M) et en deux parties de bordure (5R1, 5R2) adjacentes qui peuvent être déplacées par rapport à ladite partie centrale (5M).

5. Dispositif selon les revendications 3 ou 4, **caractérisé en ce que** chaque pièce de bordure (5R1, 5R2) de la pièce centrale (5) de libération et de pinçage de fil est reliée à un dispositif de poussée et à un dispositif de traction (DZ1, DZ2) dont l'actionnement permet de déplacer chaque partie de bordure (5R1, 5R2) par rapport à la partie centrale (5M).

6. Dispositif selon la revendication 3 ou 4, **caractérisé en ce que** la pièce centrale (5) de libération et de pinçage de fil présente deux pièces de pinçage (K1, K2) qui sont reçues par un tube de réception (R) dans une situation inactive et subissent dans celui-ci une poussée de déplacement dirigée vers l'extérieur exercée par un dispositif mécanique, en particulier un ressort (Fe1, Fe2), et après retrait dudit tube de réception (R), sont repoussées l'une de l'autre dans une situation active.

7. Dispositif selon la revendication 6, **caractérisé en ce que** les deux pièces de pinçage (K1, K2) peuvent être rapprochées l'une de l'autre au moyen d'un dispositif de traction (Z1, Z2).

8. Dispositif selon la revendication 4, **caractérisé en ce que** lesdites parties de bordure (5T1, 5T2) peuvent être déplacées l'une par rapport à l'autre au moyen d'un dispositif de commande à coulisse (DT1, Z1, DT2, Z2, Ku1, Ku2).

9. Dispositif selon la revendication 4, **caractérisé en ce que** lesdites parties de bordure (RT1, RT2) sont reliées à la partie centrale (MT) concernée par des chambres pneumatiques/hydrauliques (Ka1, Ka2) et peuvent être déplacées par rapport à ladite partie centrale (MT) sous une action pneumatique/hydraulique.

10. Dispositif selon l'une des revendications 1 à 9, **caractérisé en ce que** la pièce centrale (5) de libération et de pinçage de fil a une région extérieure de configuration concave (Ak) destinée à être placée sur la bordure de ladite paroi d'enveloppe, de ballon, ou de surface ou selon le cas paroi de vaisseau.
